# EUROPEAN PATENT APPLICATION

(11) **EP 1 571 222 A2**
(43) Date of publication of application: **07.09.2005**
(21) Application number: 04257918.5
(22) Date of filing: 17.12.2004
(51) Int. Cl.: C12N 15/85, C07K 14/46, A01K 67/027, C12N 15/11

(54) **Fluorescent genetic fragments and the correspondant transgenic fish**

(30) Priority: 03.03.2004 US 791536
(71) Applicant: Taikong Corporation, Taipei (TW)
(72) Inventor: Lin, Shiue Lian, Taipei (TW); Chen, Yin Chun, Taipei (TW)
(74) Representative: Sexton, Jane Helen

(57) **Abstract**

The present invention relates to novel gene fragments, method of generating transgenic fish from said gene fragments, new transgenic fish generated thereof. The present invention further relates to a novel method of generating new ornamental fish by breeding transgenic fish with fish with different phenotype or behavior pattern. The resulting transgenic fish shows different phenotype and behavior pattern from each of its parents. The present invention also provides the new ornamental fish generated from said method.

## Description

### Field of the Invention

The invention relates to novel nucleic acid fragments, novel transgenic medaka and novel transgenic zebrafish. The invention also relates to a method of generating new ornamental fish from transgenic fluorescence fish and the new ornamental fish generated therefrom.

### Description of the Prior Art

Transgenic technology has become an important tool for the study of gene function. There are many ways of introducing a foreign gene into fish, including: microinjection, electroporation, sperm-mediated gene transfer, gene bombardment or gene gun, liposome-mediated gene transfer and the direct injection of DNA into muscle tissue (Xu et al., *DNA Cell Biol*. 1999. 18,85-95).

Transgenic fish have also been generated for entertainment. For example, transgenic fish expressing green fluorescence by introduction of a GFP gene fused with a fish-specific gene promoter into fertilized eggs, has been generated using medaka (Hamada, K. et al., *Mol. Marine Biol. Biotech*., 1998. 7, 173-180). Tanaka et al produced a transgenic medaka with green fluorescence in only germ cells of himedaka (Tanaka, et al, The 22nd Annual Meeting of the Molecular Biology Society of Japan, Program, Abstract, 1999. pp. 458).

Transgenic fish studies make use of genes that are driven by both heterologous and homologous sources of regulatory element, and originate from constitutive or tissue-specific expression genes. Control elements include genes from antifreeze protein, mouse metallothionein, chicken δ-crystalline, carp β-actin, salmon histone H3 and carp α-globin and so on. However, there are important drawbacks to the use of these DNA elements in transgenic fish, including low expression efficiency and the mosaic expression of transgene patterns.

The microinjection into medaka eggs of *lacZ* reporter gene driven by the medaka β-actin promoter results in the transient expression of the *lacZ* gene, even in the F1 generation, though expression is low and highly mosaic. Hamada *et al.* reported a similar result in medaka embryos derived from eggs microinjected with green fluorescence protein fused with the medaka β-actin promoter (Hamada *et al*., cited supra).

Unfortunately, conventional transgenic technologies can only produce transgenic fish emitting mosaic or weak fluorescence. The fluorescence of these transgenic fish could only be seen under the fluorescent microscope with light source at a specific wavelength. Due to the impracticality and various difficulties, these fluorescent fish species were not well received by consumers and did not achieve commercial success.

Chi-Yuan Chou *et al.* disclosed a DNA construct flanked at both ends by inverted terminal repeats (ITRs) to increase the efficient expression of transgenic genes in medaka. A uniform transgene expression was achieved in the F0 and the following two generations (Chi-Yuan Chou *et al., Transgenic Research* 2001. 10: 303-315). Although a transgenic green fluorescence medaka has been described, method and condition of generating other transgenic fish with other fluorescent protein genes (such as red fluorescent protein) is different and cannot be easily deduced from the prior art because of the different strategies of genetic construction, gene expression, gene inheritance and uncertainties of the transgenic technique.

Fish breeding has the objective to produce improved fish varieties based on the exploitation of genetic variation. Using transgenic technique to generate new ornamental fish that exhibits different fluorescent colors or phenotypic patterns one by one; however, it is apparently expensive, labor intensive and time exhausting.

The genotype of a progeny fish is the result of the combination of the genotypes of the male and female gamete, which through fusion resulted in a zygote, from which ultimately the progeny fish developed. The inventor has discovered that genetic breeding between transgenic fish with fish of the same or different species with different phenotypes or behavior patterns, such as fish with different spot patterns, stripe colors, or even susceptibility to the environmental changes, could provide a much greater choice of ornamental fish that could never have been achieved before the arrival of transgenic fish and these fish can be generated in a much faster and cheaper way than performing transgenic techniques each and every time.

### Summary of the Invention

One object of the invention is to use recombinant DNA techniques to establish a stable supply of fluorescence fish with desired transgenes.

Another object of the invention relates to a nucleic acid fragment comprising (1) a β-actin gene promoter of medaka; (2) a fluorescence gene and (3) inverted terminal repeats of adeno-associated virus; provided that the fluorescence gene is not from the gene coding green fluorescent protein.

Another object of the invention relates to a nucleic acid fragment comprising (1) an α-actin gene promoter of zebrafish; (2) a green fluorescence gene and (3) inverted terminal repeats of adeno-associated virus.

Another object of the invention relates to a nucleic acid fragment comprising (1) an α-actin gene promoter of golden zebrafish; (2) a red fluorescence gene and (3) inverted terminal repeats of adeno-associated virus.

Another object of the invention relates to plasmids comprising the fragments of the invention.

Another object of the invention relates to a method of generating a novel medaka that carry the fluorescent transgene and express fluorescent protein. In particular, the invention relates to a method of producing transgenic medaka with systemic fluorescence comprising:
(a) microinjecting the appropriate amount of the plasmid of the invention into fertilized eggs of medaka;
(b) selecting the eggs with fluorescence;
(c) hatching the selected eggs to mature;
(d) crossing the mature fish with wild-type fish; and
(e) selecting the progeny with systemic fluorescence.

Another object of the invention relates to a method of generating a novel zebrafish that carry the fluorescent transgene and express fluorescent protein. In particular, the invention relates to a method of producing transgenic zebrafish with systemic fluorescence comprising:
(a) microinjecting the appropriate amount of the plasmid of the invention into fertilized eggs of zebrafish;
(b) selecting the eggs with fluorescence;
(c) hatching the selected eggs to mature;
(d) crossing the mature fish with wild-type fish; and
(e) selecting the progeny with systemic fluorescence.

Yet another object of the present invention relates to a method of generating an ornamental fish, comprising steps of (a) generating a transgenic fish containing one or more fused fluorescent genes; (b) breeding the transgenic fish with a fish with a different phenotypes or patterns; and (c) screening for new transgenic progenies showing different phenotype or pattern from their parents. Step (a) of this method may comprise the method of the invention of producing transgenic medaka or the method of the invention of producing transgenic zebrafish.

Yet another object of the present invention relates to the new transgenic fish obtainable by the methods of the present invention.

### Brief Description of the Drawing

Figure1 illustrates the plasmid construct (a) pβ-EGFP; (b) pβ-EGFP-ITR.
Figure 2 illustrates the construction of plasmid pβ-DsRed2-1-ITR from pOBA-109 and pDsRed2-1-ITR.
Figure 3 illustrates the plasmid construct, p-αDsRedITR, with the inserted gene fragment and restriction sites.
Figure 4 shows the breeding between red TK-1 with *Oryzias curvinotus,* green TK-1 with *Oryzias curvinotus*, red TK-2 with *Brachydanio* sp, red TK-2 with *Brachydanio frankei ,* and red TK-2 with *Brachydanio rerio.*
Figure 5 shows the picture of (a) red TK-1, (b) *Oryzias curvinotus* and (c) red TK-1 x *Oryzias curvinotus*.
Figure 6 shows the picture of (a) green TK-1, (b) *Oryzias curvinotus* and (c) green TK-1 × *Oryzias curvinotus.*
Figure 7 shows the picture of (a) red TK-2, (b) *Brachydanio* sp and (c) red TK-2 × *Brachydanio sp.*
Figure 8 shows the picture of (a) red TK-2, (b) *Brachydanio frankei* and (c) red TK-2 × *Brachydanio frankei*.
Figure 9 shows the picture of (a) red TK-2, (b) *Brachydanio rerio* and (c) Purple Zebra Fish.

### Detailed Description of the Invention

### Gene fragment pβ-DsRed2-1-ITR

To overcome the disadvantages associated with the transgenic fluorescent fish in the prior art, the present invention is of thorough and careful design with conceptual breakthrough. The present invention provides a gene fragment comprising (1) a β-actin gene promoter of medaka; (2) a fluorescence gene and (3) inverted terminal repeats (ITR) of adeno-associated virus; provided that the fluorescence gene is not from the gene coding green fluorescent protein.

The present invention preferably provides a plasmid construct, pβ-DsRed2-1-ITR, generated by introducing the β-actin gene promoter of medaka into expression vector pDsRed2-1-ITR. pDsRed2-1-ITR can be generated by inserting inverse terminal repeats of adeno-associated virus into the commercially available vector pDsRed-2-1 (Clontech, USA). pDsRed2-1 encodes DsRed2, a variant of DsRed (the red fluorescent protein from *Discosoma genus*). The DsRed2 variant has been engineered for faster maturation and lower non-specific aggregation. In mammalian cell cultures in which DsRed2 is expressed constitutively, red-emitting cells can be detected by fluorescence microscopy within 24 hours of transfection.

The preferred fragment of the invention is

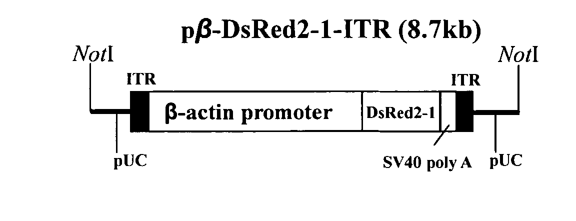

The present invention also provides a plasmid comprising the gene fragment of the invention.

### Method of generating new transgenic Medaka

The present invention also provides a method of generating new transgenic Medaka. The appropriate amount of the said plasmid, pβ-DsRed2-1-ITR, is microinjected into the cytoplasm of fertilized eggs of medaka prior to the first cleavage. These eggs are screened to find progeny expressing fluorescence throughout their systemic skeletal muscle. Progeny with fluorescent transgene are then used for future breeding.

The method of the invention provides five improvements over other methods currently available:
1. The nucleic acid fragments of the invention can be made from a commercially available vector. For instance, pβ-DsRed2-1-ITR can be made from the vector pDsRed2-1 (Clontech, USA).
2. The nucleic acid fragment of the invention enables the medaka to emit fluorescence throughout its systemic skeletal muscle.
3. The method of the invention, which comprises microinjecting the transgene construct into fertilized eggs, ensures the transgenic medaka emits fluorescence throughout its systemic skeletal muscle at a higher ratio with better quality.
4. The heterologous transgenic fish stably pass the transgene to the next generation. Thus natural breeding could be used to maintain the transgenic population and reduce the breeding cost.
5. The fluorescence of the transgenic medaka, which is emitted by its systemic skeletal muscle, can be easily seen by the naked eye. The fluorescence is further intensified under a light source of shorter wavelength, providing a higher entertainment value to ornamental fish.

Given the above, the present invention provides a method of producing transgenic medaka comprising:
a. microinjecting the appropriate amount of the above plasmid of the present invention into fertilized eggs of medaka;
b. selecting the eggs with fluorescence;
c. hatching the selected eggs to mature;
d. crossing the mature fish with wild-type fish; and
e. selecting the progeny with systemic fluorescence.

The preferred fluorescent gene used in the method of the invention is the red fluorescent gene from the vector pDsRed2-1 (Clontech, USA).

In the method of the invention of producing transgenic medaka, the appropriate amount of Notl-linearized of the invention plasmid construct injected into the fertilized eggs is typically sufficient to introduce the transgene into the germ cells of the medaka. The preferred amount of linearized plasmid construct injected into the fertilized eggs is 1-10 nl. The most preferred amount of linearized plasmid construct injected into the fertilized eggs is 2-3 nl.

### Red fluorescence transgenic fish

The present invention also provides transgenic fish obtainable by the method of the invention. In particular, the present invention provides transgenic medaka generated using the expression vector pβ-DsRed2-1-ITR. More particularly the present invention provides the red fluorescence medaka "red TK-1".

Although the method of generating fluorescent Medaka expressing EGFP has been disclosed in Chi-Yuan Chou et al., cited supra, it cannot be easily deduced from the said reference to generate other transgenic Medaka expressing other fluorescent gene for reasons discussed below.

Regarding vector construction, the vector comprising EGFP coding sequence was constructed by cleaving out β-actin promoter from pOBA-109 using restriction enzyme *Sal*I and *Nco*I. The retrieved 4 kb DNA fragment was ligated to the 4.7 kb fragment of pEGFPITR vector digested with the same enzymes. The vector of the present invention, which comprises RFP coding sequence, has fewer restriction sites and is more difficult to construct. First, the β-actin promoter was cut out from plasmid pOBA-109 by using *EcoR*I and *Nco*I. The overhanging 5' ends of the digested fragments were filled in and the 4 kb fragments were collected. Plasmid pDsRedITR was digested with restriction enzyme *Sal*I and *BamH*I and the 5' terminals were dephosphorylated. The 4.7 kb digested fragment was collected. At the end, the β-actin promoter fragment was ligated to the 4.7 kb fragment.

Regarding microinjection of fertilized eggs, the capillary needle used to generate green fluorescent fish had the diameter of 4-5 µm, and the inner wall of syringe was siliconized. 20-30 pl of DNA (10 ng/ml) was injected under 100 X magnification. The survival rate of eggs after the injection was 85%-90%. The capillary needle used in the present invention has the diameter of 1.6-2.5 µm, the smallest diameter that DNA solution does not stuck the needle, and the inner wall of the syringe was not siliconized. 20-30 pl of DNA solution was injected. The survival rate after the injection was 90-95%, 5% higher than the survival rate of green fluorescent fish.

Because RFP expresses more stably than GFP, the GFP in transgenic medaka looks yellow-green and orange-green. The RFP expressed in transgenic medaka does not show color deviation. It is possible that RFP have toxicity to the embryo, since all embryos survived were heterozygotes.

The difficulty of breeding red fluorescent Medaka compared to green fluorescent medaka is shown in Table 1 below.

**Table 1.**

| The comparison of breeding green TK-1 and red TK-1. | | | | | |
|---|---|---|---|---|---|
| Genotype | | Green TK-1 | | Red TK-1 | |
| | | Heterozygous | Dominant homozygous | Heterozygous | Dominant homozygous |
| Large-scale breeding condition | Broodstock size (mm) | 30 | 30 | 30 | 28 |
| | Numbers of male fish | 90 | 90 | 90 | 90 |
| | Numbers of female fish | 90 | 90 | 90 | 90 |
| | Total eggs/breeding session | 500 | 500 | 500 | 60 |
| | Average egg numbers/ 1 fish | 5.6 | 5.6 | 5.6 | 0.7 |
| | Defected egg numbers/ breeding session | 95 | 100 | 85 | 42 |
| | Percentage of defected eggs (%) | 19% | 20% | 17% | 70% |
| | Hatched progeny numbers/ breeding session | 385 | 380 | 390 | 16 |
| | Hatched progenies percentage (hatched progenies/non-defected eggs) | 95.1% | 95.0% | 94.0% | 88.9% |
| | Progeny numbers after a month/ breeding session | 285 | 280 | 315 | 6 |
| | Survival rate (Progeny numbers after a month/hatched progeny numbers) | 73.2% | 73.4% | 80.8% | 37.5% |
| | Non-fluorescent Japan Medaka/ breeding session | 63 | 2 | 65 | 0 |
| | Numbers of Heterozygosity/ breeding session | 143 | 10 | 225 | 0 |
| | Numbers of Homozygosity/ breeding session | 79 | 268 | 25 | 6 |
| | Numbers of fish grown to 30mm/ breeding session | 210 | 200 | 185 | 2 |
| Optimum breeding condition | Total length/ fish (mm) | 36 | 36 | 36 | 28 |
| | body weight/fish (mg) | 600 | 590 | 580 | 260 |
| | Eggs weight/total egg numbers | 45 | 41 | 42 | 7.8 |
| | egg number/ fish | 36 | 32 | 35 | 7 |
| | Average egg weight (mg) | 1.3 | 1.3 | 1.2 | 1.1 |
| Three-month old progeny fish | Total length (mm) | 28 | 30 | 29 | 22 |
| | body weight (mg) | 280 | 320 | 290 | 190 |
| Maximum adult fish | Total length (mm) | 48 | 49 | 46 | 29 |
| | body weight (mg) | 1160 | 1150 | 1090 | 280 |

Table 1 compares the breeding condition of green TK-1 (disclosed in Chi-Yuan Chou et al., cited supra) and red TK-2 of the present invention. As shown in Table 1, the total eggs, the average egg numbers per fish, percentage of defected eggs, percentage of hatched progenies, survival rate of progenies, number of fish grown to 30 mm, egg numbers per fish, average egg weight, total length of adult fish and average body weight of adult fish of the red TK-1 are significantly lower than that of green TK-1. These data indicated that breeding red TK-1 is significantly more difficult than breeding green TK-1 and thus the method of the invention is not identical to the method disclosed in Chi-Yuan Chou et al, cited supra.

### Gene fragment p-αDsRedITR

The present invention provides a gene fragment comprising (1) a α-actin gene promoter of zebrafish; (2) a fluorescence gene and (3) inverted terminal repeats (ITR) of adeno-associated virus.

The present invention preferably provides a plasmid construct, p-αDsRedITR, generated by introducing the α-actin gene promoter of golden zebrafish into expression vector pDsRed2-1-ITR. PDsRed2-1-ITR can be generated by inserting inverse terminal repeats of adeno-associated virus into the commercially available vector pDsRed2-1 (Clontech, USA).

The present invention also provides a plasmid construct, p-αEGFPITR, generated by introducing the α-actin gene promoter of golden zebrafish into expression vector pDsRed2-1-ITR and swapping the EGFP gene with the DsRed gene of the vector pDsRed2-1-ITR.

Accordingly, the preferred fragment of the invention is 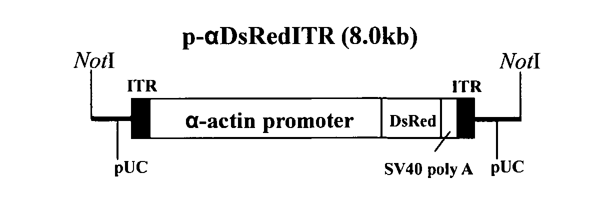 or

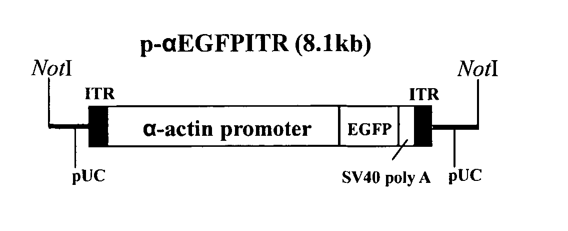

The present invention also provides a plasmid comprising the gene fragment of the invention.

The red fluorescent gene of the present invention can be purchased from BD Bioscience Clontech. In one embodiment of the invention, the vector pDsRed2-1 is used as the source of the red fluorescent gene.

### Method of generating new transgenic zebrafish

The present invention also provides a method of producing transgenic zebrafish comprising:
(a) microinjecting the appropriate amount of the above plasmid of the present invention into fertilized eggs of zebrafish;
(b) selecting the eggs with fluorescence;
(c) hatching the selected eggs to mature;
(d) crossing the mature fish with wild-type fish; and
(e) selecting the progeny with systemic fluorescence.

The preferred fluorescent gene used in the method of the invention is red fluorescent gene from pDsRed2-1.

The preferred zebrafish is Golden Zebra Danio (hereinafter called as golden zebrafish).

In the method of the invention of producing transgenic golden zebrafish, the appropriate amount of *Not*I-linearized plasmid construct injected into the fertilized eggs is typically sufficient to introduce the transgene into the germ cells of the golden zebrafish. The preferred amount of linearized plasmid construct injected into the fertilized eggs is 1-10 nl. The most preferred amount of linearized plasmid construct injected into the fertilized eggs is 2-3 nl.

### Red fluorescence transgenic fish

The present invention also provides transgenic fish obtainable by the method of the invention. In particular, the present invention provides transgenic zebrafish generated using the expression vector, p-αDsRedITR. More particularly the present invention provides the red fluorescence golden zebrafish "red TK-2".

### Green fluorescence transgenic fish

The present invention also provides transgenic fish generated using the expression vector, p-αEGFPITR. More particularly the present invention provides the green fluorescence golden zebrafish "green TK-2".

### Method of generating new ornamental fish

The present invention is provided with a method for generating an ornamental fish, comprising steps of (a) generating a transgenic fish containing one or more fused fluorescent genes; (b) breeding the transgenic fish with a fish with a different phenotype or pattern; and (c) screening the new transgenic progenies showing different phenotypes or patterns from their parents.

As used herein, ornamental fish refers to transgenic fish, or progeny of a fish, into which an exogenous construct has been introduced. A fish into which a construct has been introduced includes fish that have developed from embryonic cells into which the construct has been introduced. As used herein, an exogenous construct is a nucleic acid that is artificially introduced, or was originally artificially introduced, into a fish. The term artificial introduction is intended to exclude introduction of a construct through normal reproduction or genetic crosses. That is, the original introduction of a gene or trait into a line or strain of animal by cross breeding is intended to be excluded. However, fish produced by transfer, through normal breeding, of an exogenous construct (that is, a construct that was originally artificially introduced) from a fish containing the construct are considered to contain an exogenous construct. Such fish are progeny of fish into which the exogenous construct has been introduced.

As used herein, progeny of a fish are any fish which are descended from the fish by sexual reproduction or cloning, and from which genetic material has been inherited. In this context, cloning refers to production of a genetically identical fish from DNA, a cell, or cells of the fish. The fish from which another fish is descended is referred to as a progenitor fish. As used herein, development of a fish from a cell or cells (embryonic cells, for example), or development of a cell or cells into a fish, refers to the developmental process by which fertilized egg cells or embryonic cells (and their progeny) grow, divide, and differentiate to form an adult fish.

The examples illustrate the manner in which new transgenic fish exhibiting different phenotype or patterns from its progenitor fish can be made and used. The transgenic fish described in the examples are particularly useful to increase the commercial value of ornamental fish.

### Transgene Construct

Transgene constructs are the genetic material that is introduced into fish to produce a transgenic fish. Such constructs are artificially introduced into fish. The manner of introduction, and, often, the structure of a transgene construct, renders such a transgene construct an exogenous construct. Although a transgene construct can be made up of any nucleic acid sequences, for use in the disclosed transgenic fish it is preferred that the transgene constructs combine expression sequences operably linked to a sequence encoding an expression product. The transgenic construct will also preferably include other components that aid expression, stability or integration of the construct into the genome of a fish. As used herein, components of a transgene construct referred to as being operably linked or operatively linked refer to components being so connected as to allow them to function together for their intended purpose.

### Fish

The disclosed constructs and methods can be used with any type of fish. As used herein, fish refers to any member of the classes collectively referred to as pisces. It is preferred that fish belonging to species and varieties of fish of commercial or scientific interest be used. These fish include Cichlidae (such as *Pseudotropheus, Cichlasoma, Apistogramma, Pterophyllum* or *Symohysodon*), Fighting fish (such as *Betta* or *Macropodus*), Catfish (such as *Corydoras,* *Ancistrus* or *Pterygoplichthys*), Characidae (such as Tetras or *Carnegiella*)*,* Cyprinidae (such as *Cyprinus, Brachydanio* (zebrafish), *Danio* or *Carassius*) and Killifish (such as Medaka, Rivulines or Livebearing Toothcarps).

The preferred fish for use with the disclosed constructs and methods is medaka and zebrafish (*Brachydanio* or *Danio*). Medaka and zebrafish have significant advantages to use for ornamental fish that they are largely transparent (Kimmel, *Trends Genet* 5:283-8 (1989)). General zebrafish care and maintenance is described by Streisinger, *Natl. Cancer Inst. Monogr*. 65:53-58 (1984). The term "medaka" in the invention is not limited but to that from Adrianichthyidae (Ricefishes) such as *Oryzias javanicus, Oryzias latipes, Oryzias nigrimas, Oryzias luzonensis, Oryzias profundicola, Oryzias matanensis, Oryzias mekongensis, Oryzias minutillus, Oryzias melastigma, O.curvinotus, O. celebensis, Xenopecillus oophorus,* and *Xenopecillus saracinorum*. The preferred medaka is not limited but to that from Oryziinae such as *Oryzias javanicus, Oryzias latipes, Oryzias nigrimas, Oryzias luzonensis, Oryzias profundicola, Oryzias matanensis, Oryzias mekongensis, Oryzias minutillus, Oryzias melastigma, O.curvinotus, O. celebensis.* The most preferred medaka is *Oryzias latipes.*

The preferred Brachydanio is selected from the group consisting of *D. acrostomus, D. aequipinnatus, D. malabaricus, D. albolineatus, D. annandalei, D. apogon, D. apopyris, D. assamensis, D. choprae, D. chrysotaeniatus, D. dangila, D. devario, D. fangfangae, D. frankei, D. fraseri, D. gibber, D. interruptus, D. kakhienensis, D. kyathit, D. laoensis, D. leptos, D. maetaengensis, D. malabaricus, D. naganensis, D. neilgherriensis, D. nigrofasciatus, D. pathirana, D. regina, D. rerio, D. roseus, D. salmonata, D. shanensis* and *D. spinosus.* The most preferred is golden zebrafish.

Medaka and zebrafish embryos are easily accessible and nearly transparent. Given these characteristics, a transgenic medaka or zebrafish egg or embryo, carrying a construct encoding a reporter protein and systemic expression sequences, can provide a rapid real time in vivo identification of successful transgenics. Other fish with some or all of the same desirable characteristics are also preferred.

### Fluorescent Gene

In the methods and products of the invention, the fluorescent gene encodes a protein selected from the group consisting of green fluorescent protein (GFP), modified green fluorescent protein, enhanced green fluorescent protein (EGFP), red fluorescent protein (RFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP), and enhanced cyan fluorescent protein (ECFP). The preferred fluorescent gene is selected from the group consisting of genes encoding green fluorescent protein (GFP), modified green fluorescent protein, enhanced green fluorescent protein (EGFP), red fluorescent protein (RFP) and enhanced red fluorescent protein (ERFP). The fluorescence gene preferably encodes DsRed2.

### Generation of the Progenitor Transgenic Fish

The disclosed transgenic fish are produced by introducing a transgene construct into cells of a fish, preferably embryonic cells, and most preferably in a single cell embryo. Where the transgene construct is introduced into embryonic cells, the transgenic fish is obtained by allowing the embryonic cell or cells to develop into a fish. Introduction of constructs into embryonic cells of fish, and subsequent development of the fish, are simplified by the fact that embryos develop outside of the parent fish in most fish species.

The disclosed transgene constructs can be introduced into embryonic fish cells using any suitable technique. Many techniques for such introduction of exogenous genetic material have been demonstrated in fish and other animals. These include microinjection, electroporation, particle gun bombardment, and the use of liposomes. The preferred method for introduction of transgene constructs into fish embryonic cells is by microinjection.

Embryos or embryonic cells can generally be obtained by collecting eggs immediately after they are laid. Depending on the type of fish, it is generally preferred that the eggs be fertilized prior to or at the time of collection. This is preferably accomplished by placing a male and female fish together in a tank that allows egg collection under conditions that stimulate mating. After collecting eggs, it is preferred that the embryo be exposed for introduction of genetic material by removing the chorion. This can be done manually or, preferably, by using a protease such as pronase. A fertilized egg cell prior to the first cell division is considered a one cell embryo, and the fertilized egg cell is thus considered an embryonic cell.

After introduction of the transgene construct the embryo is allowed to develop into a fish. This generally involves incubating the embryos under the same conditions used for incubation of eggs. However, the embryonic cells can also be incubated briefly in an isotonic buffer. If appropriate, expression of an introduced transgene construct can be observed during development of the embryo.

Fish harboring a transgene can be identified by any suitable means. For example, the genome of potential transgenic fish can be probed for the presence of construct sequences. To identify transgenic fish actually expressing the transgene, the presence of an expression product can be assayed. Several techniques for such identification are known and used for transgenic animals and most can be applied to transgenic fish. Probing of potential or actual transgenic fish for nucleic acid sequences present in or characteristic of a transgene construct is preferably accomplished by Southern or Northern blotting. Also preferred is detection using polymerase chain reaction (PCR) or other sequence-specific nucleic acid amplification techniques. Transgenic medaka and zebrafish that fluoresce because of the transgene they carry can be directly observed with eyes and are described in the examples.

### Generating the new transgenic fish

The invention relates to a process for the production of haploid cells and to the production of the new transgenic fish from these cells.

According to the invention haploid cells are preferably derived from a meiotic process. The process of meiosis forms the pivotal event in the life cycle of living organisms at which genetic variation is created. Moreover it marks the transition between the diploid and the haploid. The specialized cell in the female reproductive organ that enters meiosis, which is called oocyte cell, is embedded in the differentiated ovule inside the ovary. The oocytes go through meiosis and generate haploid eggs.

Within the male reproductive tissues, a similar process leads to the formation of haploid sperms.

Although there are significant differences in the cellular processes leading to the formation of female and male gametes, the cytological events that occur during female and male meiosis are very similar suggesting the involvement of common gene products.

The end product of meiosis is a set of four genetically distinct haploid cells, which can undergo mitosis to develop into gametes. The gamete, which upon fusion leads to the formation of a zygote, develops into an embryo that can grow out the next generation.

In order to generate the new transgenic fish, the progenitor fish carrying the transgene are bred with the other progenitor fish with different phenotype or patterns. The phenotype of the fish is selected from the group consisting of colors, fluorescent colors, body shapes, body sizes, body transparent levels, grain colors, stripe colors, fin shapes, fin sizes, tail shapes, tail sizes, eye colors, eye shapes; and any observable phenotypic differences from those of fluorescent mate. In the preferred embodiment, the phenotype of the fish is selected from the group consisting of colors, body shapes, body transparent levels, grain colors and stripe colors.

The pattern of the fish is selected from the group consisting of grain patterns, stripe patterns and swimming patterns.

Haploid gametes carrying the transgene from the progenitor fish were fertilized with haploid gametes from the other progenitor fish with different phenotypes of patterns. The fertilization can be done using any suitable breeding techniques available. Species-specific breeding conditions may be required. Example of generating new transgenic medaka and zebrafish is illustrated in the figures. For example, the preferred transgenic fish is TK1 red x *O.curvinotus*, TK1 green x *O.curvinotus*, TK2 red x *Brachydanio frankei*, TK2 red x *Branchydanio sp,* TK2 green x *Brachydanio frankei*, TK2 green x *Branchydanio* sp or Purple Zebra Fish.

The transgenic fish and the fish with different phenotype or pattern may be of the same or different family, genus or species. In the preferred embodiment, the transgenic fish and the fish with different phenotype or pattern are different genus or species.

Those skilled in the art will recognize, or be able to ascertain using no more than routine experimentation, many equivalents to the specific embodiments of the invention described herein. Such equivalents are intended to be encompassed by the following claims.

### Examples

The examples below are non-limiting and are merely representative of various aspects and features of the present invention.

### Example 1 Generation of the plasmid pβ-EGFPITR

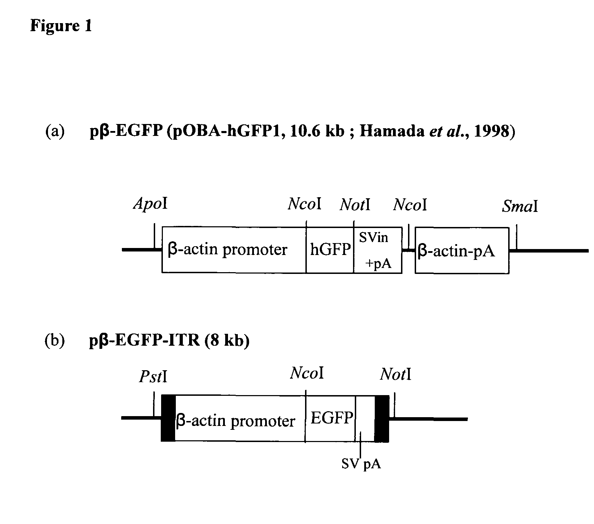

In order to generate the green fluorescent medaka, "green TK-1", plasmid pβ-EGFPITR was constructed according to Chou CY et al, *Transgenic Res.* 2001 10(4): 303-15, and are presented in Figure 1. The pβ-EGFP contained a medaka β-actin promoter fused with hGFP1 cDNA, an intron of small t antigen, SV40 polyA, and polyA from the medaka β-actin gene. The medaka β-actin promoter was obtained from pOBA-hGFP1 and was digested with *Sal*I and *Nco*I. The 3.8 kb fragment end-product was ligated to the 4.2 kb *Sall*-*Ncol* fragment from pCMV-EGFPITR. The final result was an 8 kb pβ-EGFPITR plasmid in which EGFP cDNA was driven by the β-actin promoter and flanked at both ends by AAV-ITR.

Appropriate amount of pβ-EGFPITR was restricted by *Not*I. The molecular mass for the pβ-EGFPITR DNA fragment was 7.6 kb as expected.

### Example 2 Generation of the plasmid pβ-DsRed2-1-ITR

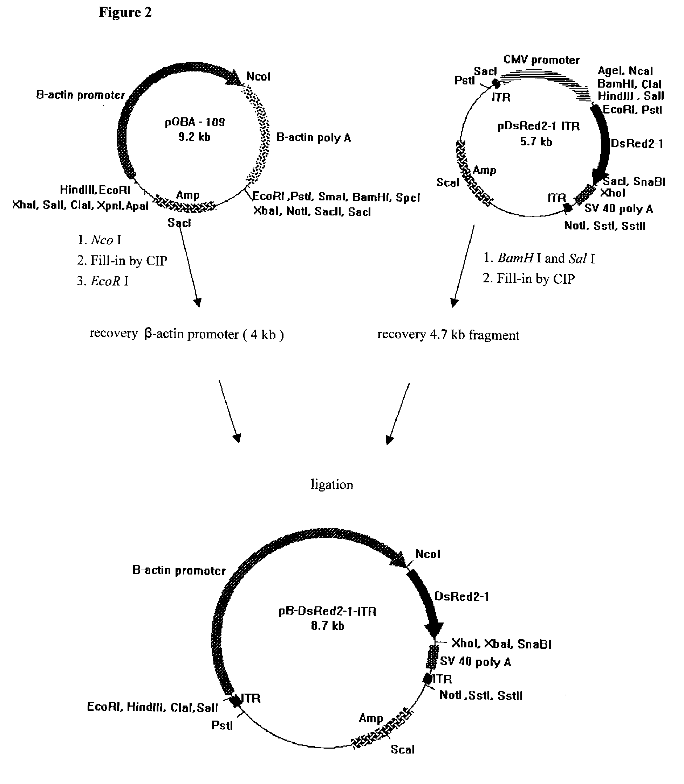

In order to generate the red fluorescent medaka, "red TK-1", plasmid pβ-DsRed2-1-ITR was constructed. As illustrated in Figure 2, the medaka β-actin gene promoter was obtained by digesting plasmid construct pOBA-109 with restriction enzymes *Ncol* and *EcoR*I. *Nco*I was used first, ends were filled in, and a subsequent digestion with *EcoR*I provided a 4 kb fragment.

As illustrated in Figure 2, the CMV promoter was cut out by digesting the construct pDsRed2-1-ITR with restriction enzymes *BamH*I and *Sal*I. Digestion with *BamH*I and *Sal*I provided a 4.7 kb fragment. Then, the β-actin gene promoter of medaka was inserted into the plasmid construct, pDsRed2-1-ITR, at the position where the CMV promoter was cut out. The resulting plasmid construct had two 145 bp adeno-associated virus inverted terminal repeats (ITR). One ITR was located at the 3' end of SV40 poly A. The other was located at the 5' end of the β-actin gene promoter.

As illustrated in Figure 2, the resulting plasmid construct, pβ-DsRed2-1-ITR, had a total length of 8.7 kb. pβ-DsRed2-1-ITR contained (1) the medaka β-actin gene promoter (for ubiquitous expression of whole body); (2) sea coral red fluorescent protein; (3) adeno-associated virus inverted terminal repeats; and (4) pUC plasmid construct basis.

Appropriate amount of pβ-DsRed2-1-ITR was digested with proportional amount of *Not* I restriction enzyme. A small fraction of the digested product was analyzed by agarose gel electrophoresis to verify its linearity. The fragment length was 8.7 kb as expected.

### Example 3 Generation of plasmid p-αEGFPITR

In order to generate the green fluorescent golden zebrafish, "green TK-2", plasmid p-αEGFPITR was constructed.

The medaka β-actin promoter of pβ-EGFPITR was cut out by digesting with restriction enzymes *Sal*I and *Nco*I. Then, the α-actin gene promoter of golden zebrafish was inserted into the plasmid construct at the position where the medaka β-actin promoter was cut out. The resulting plasmid construct had two 145 bp adeno-associated virus inverted terminal repeats (ITR). One ITR was located at the 3' end of SV40 poly A. The other was located at the 5' end of the α-actin gene promoter.

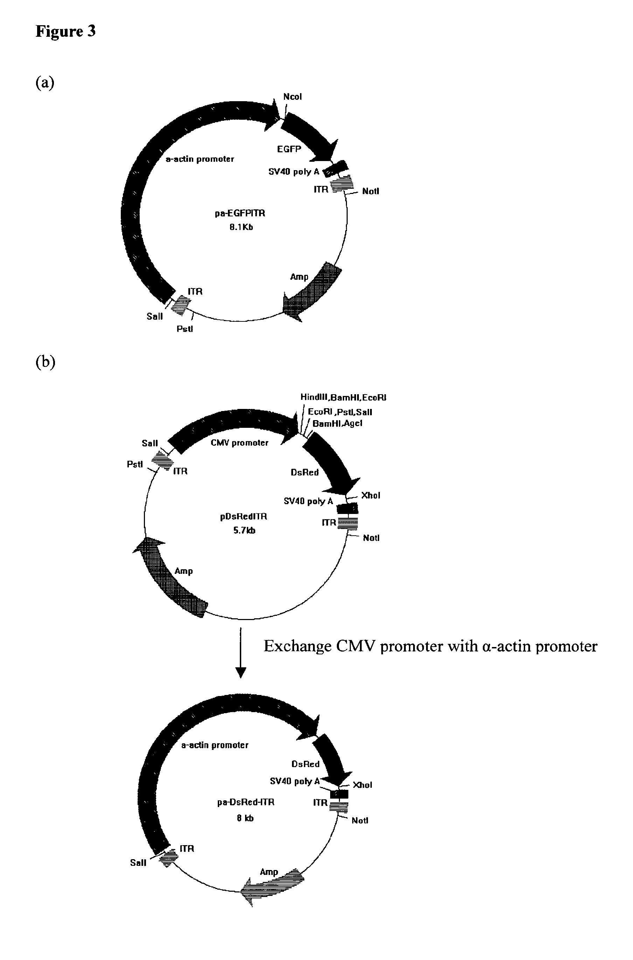

As illustrated in Figure 3(a), the resulting plasmid construct, p-αEGFPITR, had a total length of 8.1 kb. p-αEGFPITR contained (1) the golden zebrafish α-actin gene promoter (for ubiquitous expression of whole body); (2) green fluorescent protein; (3) adeno-associated virus inverted terminal repeats; and (4) pUC plasmid construct basis.

Appropriate amount of p-αEGFPITR was digested with proportional amount of *Not* I restriction enzyme. A small fraction of the digested product was analyzed by agarose gel electrophoresis to verify its linearity. The fragment length was 8.1 kb as expected.

### Example 4 Generation of plasmid p-αDsRedITR

In order to generate the red fluorescent zebrafish, "red TK-2" , plasmid p-αDsRed2-1-ITR was constructed. As illustrated in Figure 3(b), the golden zebrafish α-actin gene promoter was obtained by digesting plasmid construct p-αEGFPITR with restriction enzymes *Ncol* and *Sal*I. *Nco*l was used first, ends were filled in, and a subsequent digestion with *Sal*I provided a 3.8 kb fragment.

As illustrated in Figure 3(b), the CMV promoter was cut out by digesting the construct pDsRedITR with restriction enzymes *Bam*HI and *Sal*I. *Bam*HI was used first, ends were filled in, and a subsequent digestion with *Sal*I provided a 4.2 kb fragment. Then, the α-actin gene promoter of golden zebrafish was inserted into the plasmid construct, pDsRedITR, at the position where the CMV promoter was cut out. The resulting plasmid construct had two 145 bp adeno-associated virus inverted terminal repeats (ITR). One ITR was located at the 3' end of SV40 poly A. The other was located at the 5' end of the α-actin gene promoter.

As illustrated in Figure 3(b), the resulting plasmid construct, p-αDsRedITR, had a total length of 8.0 kb. The resulting construct p-αDsRedITR contained (1) the golden zebrafish α-actin gene promoter (for systemic gene expression); (2) sea coral red fluorescent protein; (3) adeno-associated virus inverted terminal repeats; and (4) pUC plasmid construct basis.

Appropriate amount of p-αDsRedITR was digested with proportional amount of *Not* I restriction enzyme. A small fraction of the digested product was analyzed by agarose gel electrophoresis to verify its linearity. The fragment length was 8 kb as expected.

### Example 5 Preparation of Microinjected DNA

All DNA plasmids were prepared via ultra-centrifugation with cesium chloride and ethidium bromide gradient (Radloff et al., 1967 *Proc Natl Acad Sci USA* 57:1514-1521). All DNA fragments used for microinjection were eluted from agarose gel following electrophoresis.

### Example 6 Cytoplasmic Microinjection

The procedures followed for cytoplasmic microinjection are described in detail in Kinoshita and Ozato, 1995 *Fish Biol J MEDAKA* 7:59-64 and Kinoshita et al. 1996 *Aquaculture* 143:267-276.

Fish were maintained under artificial conditions of 14 h light and 10 h darkness at 26°C and maintained on a diet of Tetramin (Tetra, Germany). Before the incubator entered the dark cycle, fish were collected and separated by separation net. On the next morning after the light cycle has begun, fish eggs were collected every 15-20 minutes.

Eggs were collected within 30 min of fertilization and attaching filaments removed. The linearized construct was quantified and dissolved in 5X PBS with phenol red at the desired concentration. DNA was picked up by micro-capillary of zebrafish microinjector (Drummond) wherein the injection needle width of the micro-capillary was approximately 10 µm. As micro-needle enters the cell cytoplasm, the DNA injected was approximated 2-3 nl. In each microinjection session, 30-40 eggs were injected; 250-300 eggs were injected in each experiment. Injected eggs were incubated at 26°C in distilled water.

### Example 7 Hatching and Screening for Transgenic Embryos

Injected eggs were rinsed with sterilized solution, cultured in incubator wherein the temperature was 28.5°C. The fluorescence could be observed in the developing embryo after 24 hours. Embryos were observed under a bright field with a dissecting stereomicroscope (MZAPO, Leica, Germany). Dark field illumination for detecting green fluorescence was performed with a stereomicroscope equipped with a GFP Plus filter (480 nm). The distribution and intensity of the red fluorescence is observed under fluorescence microscope (Leica MZ-12; Fluorescence System: light source Hg 100 W; main emission wavelength 558 nm, and main absorption wavelength 583 nm, filter set RFP-Plus; photography system MPS60). Photographs were taken using an MPS60 camera loaded with ISO 400 film and equipped with a controller for film exposure time (Leica, Germany). In order to examine the distribution of GFP or RFP expression in the tissues of transgenic fish, 11 days of post-fertilization larva which having GFP or RFP expression on appearance were sectioned and observed under fluorescent microscopy. Larva were fixed for 30min in 4% paraformaldehyde at 4°C, embedded in cryomatrix (Shandon, USA) and frozen at -20°C. Cryostat sections (Cryostat Microtome, HM500 OM, Microm, Germany) with 15µm thickness were mount on slides and observed the GFP or RFP fluorescence immediately.

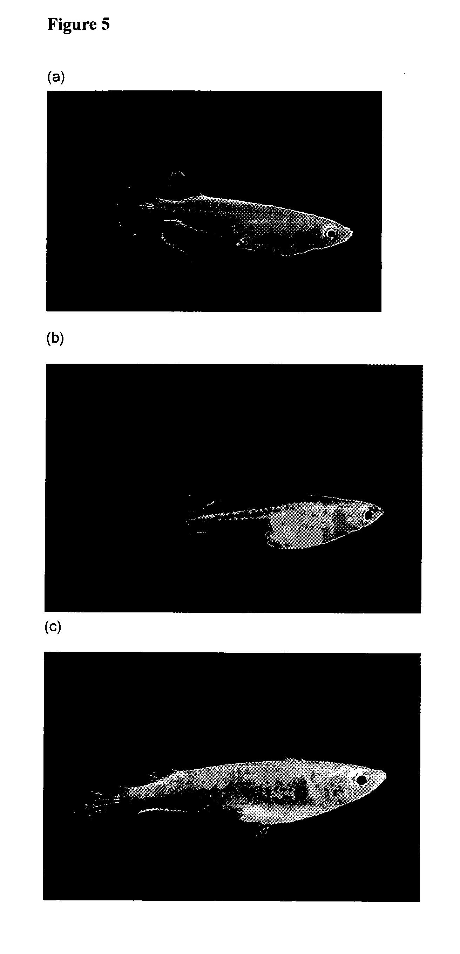

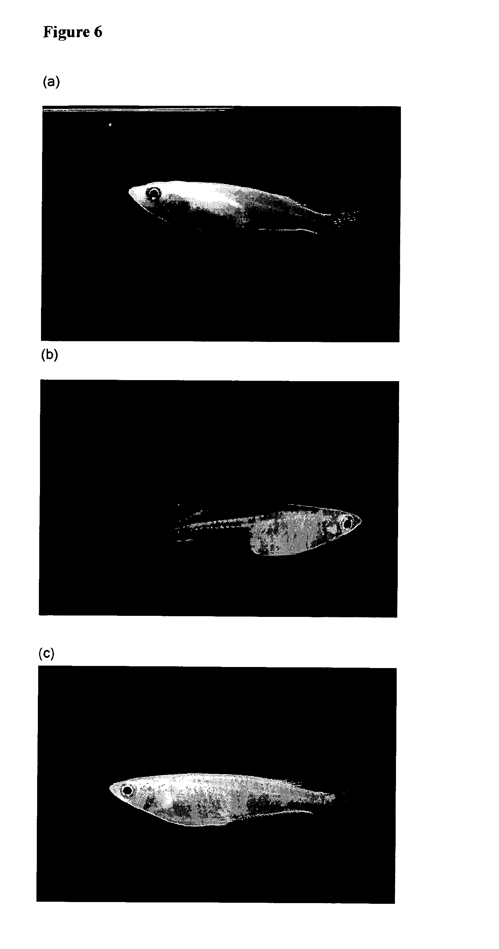

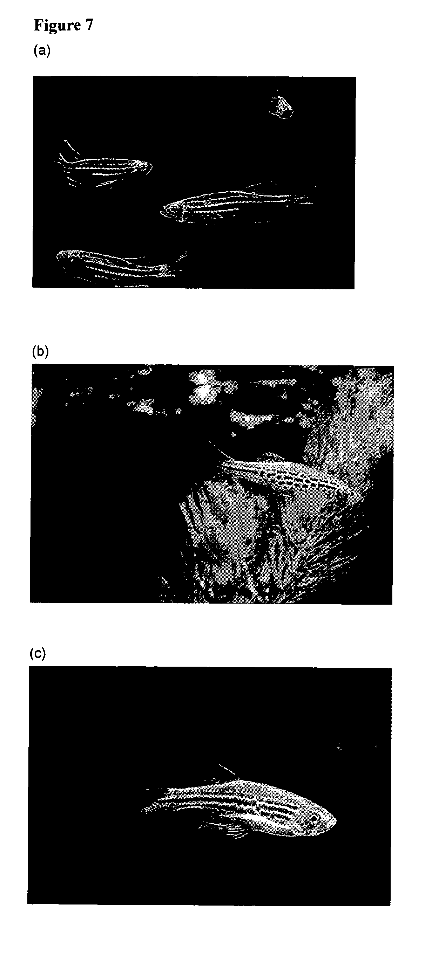

The red fluorescence fish generated from expression vector pβ-DsRed2-1-ITR is red TK-1 (Figure 5(a)). The green fluorescence fish generated from expression vector pβ-EGFPITR is green TK-1 (Figure 6(a)). The green fluorescence fish generated from expression vector p-αEGFPITR is green TK-2. The red fluorescence fish generated from expression vector p-αDsRedITR is red TK-2 (Figure 7(a)).

### Example 8 Generation of new transgenics

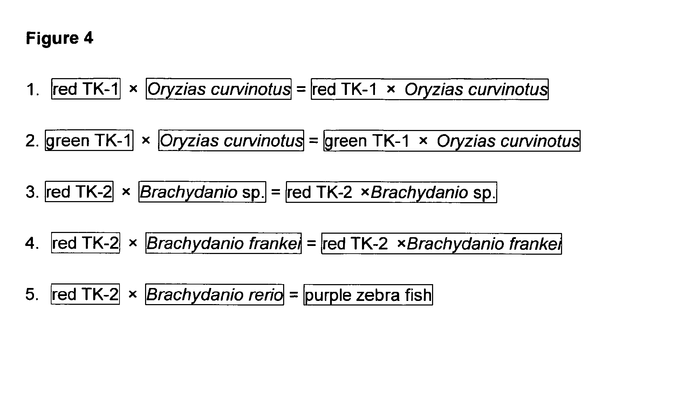

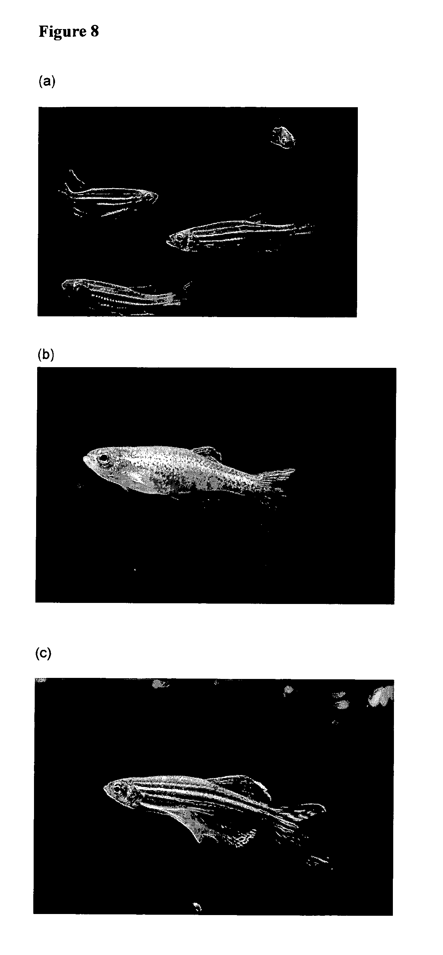

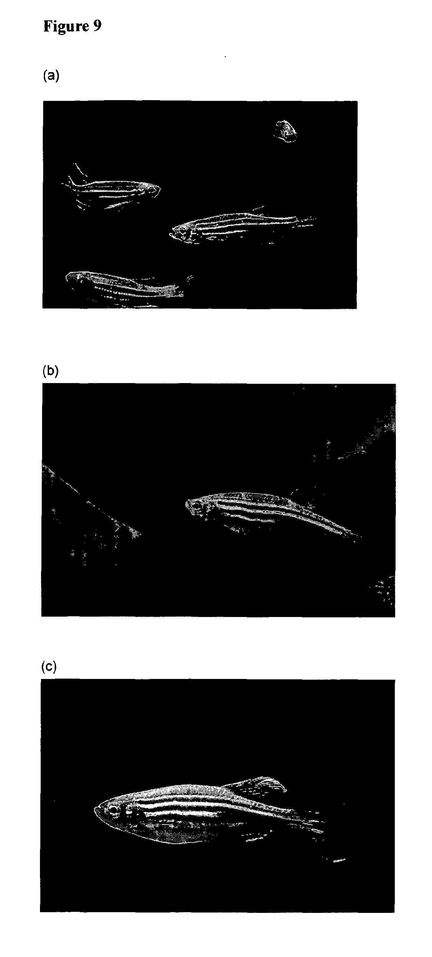

As shown in Figure 4, genetic breeding was performed between red TK-1 (Fig 5(a)) with *Oryzias curvinotus* (Fig 5(b)), green TK-1 (Fig 6(a)) with Oryzias *curvinotus* (Fig 6(b)), red TK-2 (Fig 7(a)) with *Brachydanio* sp (Fig 7(b)), red TK-2 (Fig 8(a)) with *Brachydanio frankei* (Fig 8(b)), red TK-2 (Fig 9(a)) with *Brachydanio rerio* (Fig 9(b)).

The resulting F1 progeny transgenics, namely red TK-1 × *Oryzias curvinotus*(Fig 5(c)), green TK-1 × Oryzias *curvinotus* (Fig 6(c)), red TK-2 × *Brachydanio* sp (Fig 7(c)), red TK-2 × *Brachydanio frankei* (Fig 8(c)), Purple Zebra Fish (Fig 9(c)), are products of the genetic varieties of their parents. In these examples, one of the progenitor fish exhibited green or red fluorescence whereas the other showed a specific stripe or grain pattern. The new trasngenics generated from the breeding thus inherited both characteristics and showed phenotypes that are different from each of the parent fish.

These new transgenics are self-crossed to generate F2 homozygotes and hatched as described in example 7. All of the progenies of the F2 homozygotes will inherit the transgene and show the new phenotypes.

While the invention has been described and exemplified in sufficient detail for those skilled in this art to produce and use it, various alternatives, modifications, and improvements should be apparent without departing from the spirit and scope of the invention.

One skilled in the art readily appreciates that the present invention is well adapted to carry out the objects and obtain the ends and advantages mentioned, as well as those inherent therein. The cell lines, embryos, animals, and processes and methods for producing them are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations, which are not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth within the following claims.

## Claims

1. A gene fragment comprising (1) a β-actin gene promoter of medaka; (2) a fluorescence gene; and (3) inverted terminal repeats (ITR) of adeno-associated virus; provided that the fluorescence gene is not from the gene coding green fluorescent protein.

2. The gene fragment of claim 1, wherein the fluorescence gene is red fluorescent protein (RFP).

3. The gene fragment of Claim 1 or 2 which is pUC-ITR-β-actin promoter-DsRed 2-1-SV40 poly A-ITR-pUC (pβ-DsRed2-1-ITR)

4. A plasmid comprising the gene fragment of any one of Claims 1-3.

5. A method of producing transgenic medaka with systemic fluorescence comprising:
(a) microinjecting the appropriate amount of the plasmid of Claim 4 into fertilized eggs of medaka;
(b) selecting the eggs with fluorescence;
(c) hatching the selected eggs to mature;
(d) crossing the mature fish with wild-type fish; and
(e) selecting the progeny with systemic fluorescence.

6. The method of Claim 5, wherein the appropriate amount of the plasmid injected into the fertilized eggs is sufficient to introduce transgene into germ cell of medaka.

7. The method of claim 5 or 6, wherein the medaka is *Oryzias javanicus, Oryzias latipes, Oryzias nigrimas, Oryzias luzonensis, Oryzias profundicola, Oryzias matanensis, Oryzias mekongensis, Oryzias minutillus, Oryzias melastigma, O.curvinotus, O. celebensis, Xenopecillus oophorus* or *Xenopecillus saracinorum*.

8. A transgenic medaka with systemic fluorescence obtainable by the method of Claim 5 or 6.

9. A gene fragment comprising (1) an α-actin gene promoter of golden zebrafish; (2) a fluorescence gene and (3) inverted terminal repeats (ITR) of adeno-associated virus.

10. The fragment of Claim 9 which is pUC-ITR-x-actin promoter-DsRed-SV40 poly A-ITR-pUC (p-αDsRedITR) or pUC-ITR-α-actin promoter-EGFP-SV40 poly A-ITR-pUC (p-αEGFPITR)

11. A plasmid comprising the gene fragment of Claim 10.

12. A method of producing transgenic zebrafish with systemic fluorescence comprising:
(a) microinjecting the appropriate amount of the plasmid of Claim 11 into fertilized eggs of zebrafish;
(b) selecting the eggs with fluorescence;
(c) hatching the selected eggs to mature;
(d) crossing the mature fish with wild-type fish; and
(e) selecting the progeny with systemic fluorescence.

13. The method of claim 12, wherein the zebrafish a golden zebrafish.

14. A zebrafish with systemic fluorescence obtainable by the method of Claim 12 or 13.

15. The zebrafish of Claim 14 that has systemic red fluorescence or systemic green fluorescence.

16. A method for generating an ornamental fish, comprising steps of (a) generating a transgenic fish containing one or more fused fluorescent genes; (b) breeding the transgenic fish with a fish with a different phenotype or pattern; and (c) screening the new transgenic progenies showing different phenotypes or patterns from their parents.

17. The method of claim 16, wherein step (a) comprises the method of claim 5 or 12.

18. The method of claim 16 or 17, wherein the transgenic fish and the fish with different phenotype or pattern are different genus or species.

19. The method of any one of claims 16-18, wherein the fluorescent gene is selected from the group consisting of green fluorescent protein (GFP), modified green fluorescent protein, enhanced green fluorescent protein (EGFP), red fluorescent protein (RFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP), and enhanced cyan fluorescent protein (ECFP).

20. The method of any one of claims 16-19, wherein the phenotype of the fish is selected from the group consisting of colors, fluorescent colors, body shapes, body sizes, body transparent levels, grain colors, stripe colors, fin shapes, fin sizes, tail shape, tail sizes, eye color, eye shapes; and any observable phenotypic differences from those of fluorescent mate.

21. The method of any one of claims 16-19, wherein the pattern of the fish is selected from the group consisting of grain patterns, stripe patterns and swimming patterns.

22. The method of any one of claims 16-21, wherein the fish is selected from the group consisting of Cichlidae, Fighting fish, Catfish, Characidae, Cyprinidae and Killifish.

23. The method of claim 23, wherein
(i) the Cichlidae is *Pseudotropheus, Cichlasoma, Apistogramma, Pterophyllum* or *Symohysodon;*
(ii) the Fighting fish is *Betta* or *Macropodus;*
(iii) the Catfish is *Corydoras, Ancistrus* or *Pterygoplichthys;*
(iv) the Characidae is Tetras or *Carnegiellaor;*
(v) the Cyprinidae is *Cyprinus,* Brachydanio(zebrafish), *Danio* or *Carassius;* or
(vi) the Killifish is Medaka, Rivulines or Livebearing Toothcarps.

24. The method of claim 23, wherein
(a) the zebrafish is selected from the group consisting of *D. acrostomus, D. aequipinnatus, D. malabaricus, D. albolineatus, D. annandalei , D.* *apogon, D. apopyris , D. assamensis, D. choprae, D. chrysotaeniatus, D. dangila, D. devario, D. fangfangae, D. frankei , D. fraseri, D. gibber, D. interruptus, D. kakhienensis, D. kyathit, D. laoensis, D. leptos, D. maetaengensis, D. malabaricus, D. naganensis, D. neilgherriensis, D. nigrofasciatus, D. pathirana, D. regina, D. rerio, D. roseus, D. salmonata, D. shanensis D. spinosus, Brachydanio frankei* and *Branchydanio sp; or*
(b) the medaka is selected from the group consisting of *Oryzias javanicus, Oryzias latipes, Oryzias nigrimas, Oryzias luzonensis, Oryzias profundicola, Oryzias matanensis, Oryzias mekongensis, Oryzias minutillus, Oryzias melastigma, O.curvinotus, O. celebensis. Xenopecillus oophorus,* and *Xenopecillus saracinorum*.

25. The method of any one of claims 16-24, wherein the new transgenic progenies is TK1 red x *O.curvinotus,* TK1 green x *O.curvinotus,* TK2 red x *Brachydanio frankei,* TK2 red x *Branchydanio sp,* TK2 green x *Brachydanio frankei,* TK2 green x *Branchydanio sp* or Purple Zebra Fish.

26. An ornamental fish obtainable by the method of any one of claims 16-25.
